# EUROPEAN PATENT SPECIFICATION

(11) **EP 0 227 162 B1**
(45) Date of publication and mention of the grant of the patent: **22.09.1993**
(21) Application number: 86202163.1
(22) Date of filing: 03.12.1986
(51) Int. Cl.: C08F 8/30, C08F 12/32, C07D 213/22, C07D 471/04

(54) **Complex-forming polymers**
Komplexbildende Polymere
Polymères complexants

(30) Priority: 23.12.1985 GB 8531625
(43) Date of publication of application: 01.07.1987
(73) Proprietor: SHELL INTERNATIONALE RESEARCH MAATSCHAPPIJ B.V., 2596 HR Den Haag (NL)
(72) Inventor: Stapersma, Johan, NL-1031 CM Amsterdam (NL)

(56) References cited:
- EP-A- 0 109 915
- CHEMICAL ABSTRACTS, vol. 95, no. 22, 30th November 1981, page 385, abstract no. 192884x, Columbus, Ohio, US; R.S. DRAGO et al.: "An investigation of unique metal complexes produced using polystyrene-2,2'-bipyridine as a support, and several hydrogenation catalysts derived from this copolymer", & Gov. Rep. Announce Index (US) 1981, 81(16), 3348
- CHEMICAL ABSTRACTS, vol. 98, no. 6, 7th February 1983, page 176, abstract no. 37646q, Columbus, Ohio, US; M. KANEKO et al.: "Photoresponsive graphite electrode coated with a film of polymer with pendant tris(2,2'-bipiridyl)ruthenium(II) complex", & Makromol. Chem., Rapid Commun., 1982, 3(11), 769-72

## Description

The present invention relates to nitrogen-containing complex-forming polymers, more in particular to polymers carrying 2,2′-bipyridine- or 1,10-phenanthroline-based complex-forming moieties, to the preparation of such complex-forming polymers, to novel compounds formed as intermediates in the preparation of the complex-forming polymers, and to the complexed polymers.

Nitrogen-containing complex-forming polymers are known. US patent specification 3,810,888 describes complex-forming polymers wherein 2,2′-bipyridine- or 1,10-phenanthroline-based complex-forming moieties are linked via an acetal group to the macromolecular polymer chain of a hydroxyl group-carrying polymer, which complex-forming polymers are the reaction products of a polyvinyl alcohol and 4-(p-formylstyryl)-2,2′-bipyridine or 4-(p-formylstyryl)-1,10-phenanthroline respectively. In the Journal of the American Chemical Society, 97 (12), 3454 (1975), polymeric phenanthrolines are described which are prepared by reacting a chlorosulphonated, highly cross-linked polystyrene resin with 5-amino-o-phenanthroline. The hereinbefore mentioned complex-forming polymers have in common that the complex-forming moiety is linked to the macromolecular polymer chain via a hydrolytically unstable group.

European patent application 0 045 277 describes complex-forming polymers which have pendant bipyridyl groups as complex-forming moieties, which groups are linked directly to the macromolecular polymer chain and which polymers are prepared by polymerizing a 4- or 6-vinyl-2,2′-bipyridine. These complex-forming polymers have a disadvantage in that the complex-forming moieties are situated very close to the polymer chain and hence may be inaccessible or insufficiently mobile.

In Inorganic Chemistry, 17 (9), 2345 (1978), polystyryl bipyridines are described, which complex-forming polymers are prepared by lithiating a brominated crosslinked polystyrene and subsequently reacting the lithiated polystyrene with bipyridine. It is reported that just over 50% of the brominated phenyl groups are converted to the corresponding phenylpyridine group. Said process not only has a low yield but moreover may give rise to the formation of multicoupled complex-forming groups.

In European patent application No. 109,915 there is disclosed a process for the functionalization of polymeric resins, wherein a halo-methylated resin, with a crosslink density of at least 4 per cent is reacted with an alkyllithium wherein the alkyl radical is 1 to 12 carbon atoms; followed by a reaction to produce a polymeric material having a desired pendant functionality.

This process results in a functionalized resin, wherein the pendant functionality is relatively immobile due to its closeness to the polymer chain. Besides, the applicability of the process is somewhat limited, as it can only be used for resins having a certain crosslink density. Accordingly, there is still a demand for a more versatile process, which process yields complex-forming polymers having highly mobile pendant complex-forming moieties.

Surprisingly a novel class of complex-forming polymers has now been synthesized wherein the 2,2,'-bipyridine- or 1,10-phenanthroline-based complex-forming moieties are situated at some distance from the macromolecular polymer chain and are linked to said polymer chain via a hydrolytically stable group, and which complex-forming polymers may be non-crosslinked or crosslinked polymers.

The invention provides therefore a polymer having at least one pendant aryl group carrying a complex-forming moiety of general formula 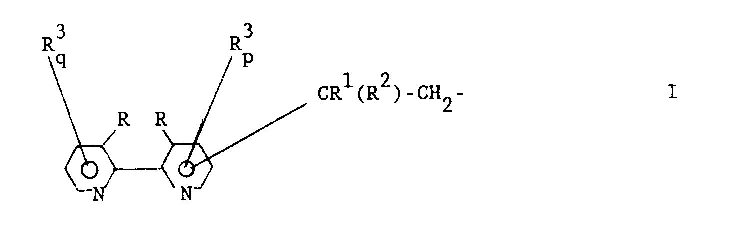
wherein each R individually is H, or both R's together form a group -CH=CH-; R¹ is H or an alkyl group; R² is H, an alkyl or a vinyl-benzyl group or a group 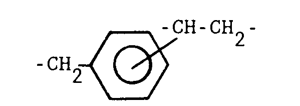
wherein the moiety 〉CH-CH₂- forms part of a macromolecular polymer chain; or both R¹ and R² together form a group -(CH₂)ₙ- wherein n is an integer in the range of from 2 up to and including 6; each R³ individually is an alkyl, phenyl, alkoxy, phenoxy, alkylthio or phenylthio group; p and q are integers from 0 up to and including 2, and from 0 up to and including 3, respectively; the -CR¹(R²)-CH₂- group is linked to a carbon atom of the heterocyclic aromatic ring which occupies the ortho- or para-position with respect to the nitrogen atom in said heterocyclic ring, with the proviso that at least one of R¹ or R² represents H if both R's together form a group -CH=CH- and if at the same time the group -CR¹(R²)-CH₂- occupies the para-position with respect to the nitrogen atom in said heterocyclic ring.

The polymers of the present invention which have at least one pendant aryl group carrying a complex-forming moiety of general formula I, comprise both non-crosslinked and crosslinked polymers. The non-crosslinked polymers having at least one such pendant aryl group include homopolymers of monovinylaromatic compounds, copolymers of more than one monovinylaromatic compound and copolymers of at least one monovinylaromatic compound and one or more mono-ethylenically unsaturated compounds. The crosslinked copolymers include copolymers of at least one monovinylaromatic compound and at least one divinyl-unsaturated compound.

The monovinylaromatic compounds will be understood to include styrene and it analogs and homologs such as α-methylstyrene, and ringsubstituted styrenes such as vinyltoluene. Styrene is a preferred monovinylaromatic compound.

Suitable mono-ethylenically unsaturated compounds include mono-ethylenically unsaturated carboxylic acids such as acrylic acid, methacrylic acid, itaconic acid, maleic acid and fumaric acid; esters of such acids, such as methyl acrylate, ethyl acrylate, butyl acrylate, methyl methacrylate, 2-ethylhexyl acrylate and vinylpyridine.

The divinyl unsaturated compounds on which the crosslinked polymers, having at least one pendant aryl group, may be based, include compounds such as a divinylbenzene, a divinyltoluene, a divinylxylene, a divinylpyridine, divinyl ketone, divinyl sulphone; divinyl ethers such as the divinyl ether of ethylene glycol and the divinyl ether of polyethylene glycol; diesters of two moles of a monoethylenically unsaturated carboxylic acid and a diol such as ethylene glycol diacrylate, ethylene glycol dimethacrylate and polyethylene glycol diacrylate. Divinylbenzene is a preferred divinyl compound.

The preferred non-crosslinked polymers having at least one pendant aryl group are polystyrene and copolymers of styrene and one or more monoethylenically unsaturated compounds. The most preferred crosslinked polymer is polystyrene.

Preferred crosslinked polymers having at least one pendant aryl group are copolymers of styrene and divinylbenzene. Especially preferred are styrene/divinylbenzene copolymers wherein divinylbenzene is present in an amount which corresponds with about 0.5 to about 25 %w of said copolymer.

It has hereinbefore been stated that in general formula I each R individually may be H in which case the complex-forming moiety represents a 2,2′-bipyridine-based complex-forming moiety of general formula 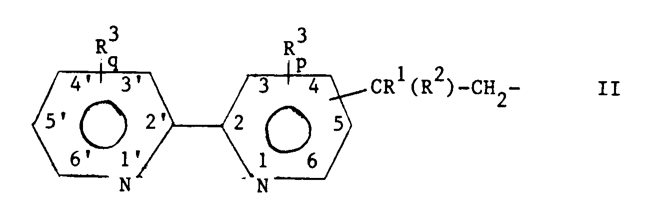
wherein R¹, R², R³, p and q have the same meaning as in general formula I. When in general formula I both R's together form a group -CH=CH- the complex-forming moiety represents a 1,10-phenanthroline-based complex-forming moiety of general formula 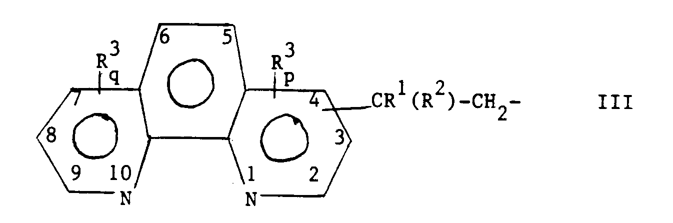
wherein R¹, R², R³, p and q have the same meaning as in general formula I. Hence in formula II the group -CR¹(R²)-CH₂- will be linked to the carbon which occupies the 4- or 6-position and in general formula III to the carbon atom which occupies the 2- or the 4-position in the heterocyclic aromatic ring.

It is preferred that in general formulae I, II or III R¹ represents H or an alkyl group having from 1 to 6 carbon atoms and that R² represents H or an alkyl group having from 1 to 6 carbon atoms, which alkyl group may be the same or different to the alkyl group R¹. It is especially preferred that when R¹ and/or R² represent an alkyl group having from 1 to 6 carbon atoms that said alkyl group is a methyl group. However, when in general formula III the -CR¹(R²)-CH₂- group is linked to the carbon atom which occupies the 4-position in the heterocyclic aromatic ring, at least one of R¹ or R² represents H.

The heterocyclic aromatic rings in general formulae I, II and/or III may be substituted by one or more alkyl, phenyl, alkoxy, phenoxy, alkylthio and/or phenylthio groups. Suitable such alkyl, alkoxy and/or alkylthio groups include those wherein the alkyl moiety is a linear or branched alkyl group, preferably having from 1 to 6 carbon atoms. Suitable such phenyl, phenyoxy and/or phenylthio groups include those wherein the phenyl group is a ringstubstituted phenyl group. The number of substituting groups R³ which may be present in general formula I, II or III is indicated by the value of the integers p, which may vary from 0 up to and including 2 and q, which may vary from 0 up to and including 3. Preferred, however, are polymers wherein in the general formulae of the complex-forming moieties p and q are both 0.

The invention also provides a process for the preparation of the hereinbefore described novel complex-forming polymers. The starting material is a compound of general formula 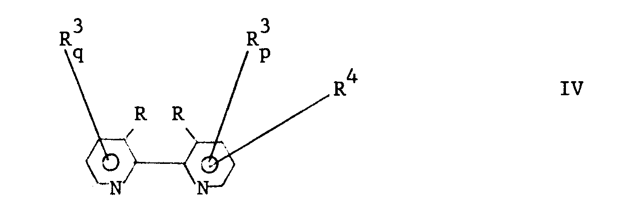
in which:
- R, R³, p and q have the same meaning as in general formula I provided that R³ is essentially inert vis-à-vis the metalating agent defined hereinafter and
- R⁴ is an alkyl group or a cycloaliphatic group having from 3 to 7 carbon atoms in the ring, which group is situated on the ortho- or para-position with respect to the nitrogen atom in said heterocyclic ring and which group has at least one hydrogen on the alpha-carbon atom (the carbon atom directly attached to said heterocyclic ring).

However, if R⁴ is situated on the para-position with respect to the nitrogen atom in said heterocyclic ring and at the same time both R-groups represent -CH=CH-, then R⁴ is an alkyl group having at least two hydrogen atoms on the alpha-carbon atom. Thus, in this case the starting material is a compound with general formula 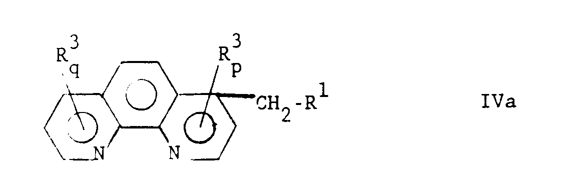
in which R¹ has the same meaning as in general formula I.

The process for the preparation of the hereinbefore described novel complex-forming polymers comprises contacting the starting material, as described by general formula IV or IVa, with a metalating agent followed by reaction of the metalated compound with either:
A.
   1. at least one vinylbenzyl halide and subsequently
   2. polymerizing the reaction product of A.1. or
B. a halomethylated polystyrene polymer.
Preferably R⁴ is an alkyl group having from 1 to 6 carbon atoms.

When comparing general formula IV with general formula I, R⁴ can be expressed in terms of R¹ and R² by general formula 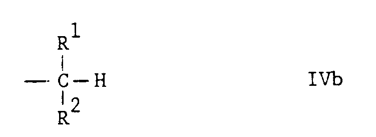
wherein R¹ and R² have the same meaning as in general formula I, except for the fact that R² in formula IVb can not represent a vinylbenzene group or a group

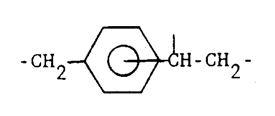

This is caused by the fact that these groups only can be introduced after the metalation step, whereas general formula IV represents the starting material, so before the metalation. The methylene group -CH₂-, which is attached to the α-carbon atom according to general formula I, has descended of the halomethylated styrene, which is added after the metalation. Thus, this group is not present in R⁴ and therefore not represented in general formula IVb.

When in general formula IV each R individually is H, general formular IV represents 2,2'-bipyridine 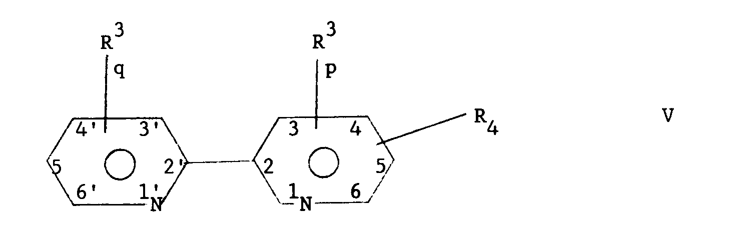
wherein R³, R⁴, p and q have the same meaning as in general formula IV and R⁴ is linked to a carbon atom which occupies the 4- or 6-position in the heterocyclic aromatic ring; when both R's form a group -CH=CH- general formula IV represents a 1,10-phenanthroline of general formula 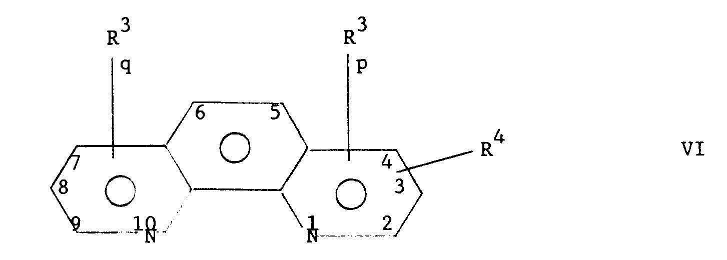
in which R³, R⁴, p and q have the same meaning as in general formula IV and R⁴ is linked to a carbon atom which occupies the 2- or 4-position in the heterocyclic aromatic ring. Although both general formulae V and VI indicate that the 2,2'-bipyridine- and/or 1,10-phenanthroline-based starting compounds may be substituted by a group R³ there is a preference for compounds of general formula V which do not have a R³-alkyl group linked to a carbon atom in the 4- or 6- and/or 4'- and/or 6'-position, or for compounds of general formula VI which do not have an R³ alkyl group linked to the carbon atom in the 2- or 4- and/or 7- and/or 9-position, as with these compounds it will not only be more difficult to metalate a specific alkyl group but moreover there is also the possibility that more than one alkyl group may be metalated. Such multi-metalated compounds may ultimately give rise to the formation of polymers wherein the complex-forming moiety is linked to macromolecular polymer chains via more that one bonding group, i.e. including a modified group R³, which could affect the mobility of said complex-forming moiety. This is distinguished from polymers wherein the complex-forming moiety is linked to more than one macromolecular polymer chain via one single bonding group ("-CR¹(R²)-CH₂-", wherein R² is a group 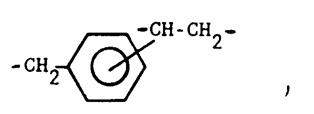
wherein the moiety 〉CH-CH₂ forms part of a polymer chain, whereby the mobility is affected to a much lesser extent.

Especially preferred are compounds of general formulae V and/or VI wherein the integers p and q are 0.

Compounds which may suitably be used in the metalation of the compounds of general formulae IV, V and/or VI include alkali metal compounds. Preferred are alkali metal amides. An especially preferred metalating compound is lithiumdiisopropylamide. The metalating compound is generally employed in a molar ratio of organometalic compound to compound of general formula V or VI of about 1 to 1 and at a temperature in the range of from about -120 °C to about 100 °C, preferably from about -80 °C to about 30 °C. The reaction is generally carried out in the presence of an inert solvent such as diethyl ether or tetrahydrofuran, mixtures thereof or mixtures thereof with suitable hydrocarbon solvents. THF is a preferred solvent.

The metalated compounds of general formulae IV, V and/or VI may subsequently be reacted with at least one vinylbenzyl halide to arrive at compounds of general formula 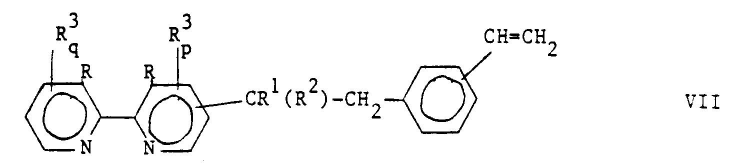
which compounds are novel, and wherein R, R¹, R², R³, p and q have the same meaning as in general formula I. Vinylbenzyl chlorides are the preferred vinylbenzyl halides. It will be understood by those skilled in the art, that the nature of vinylbenzyl halide used, i.e. o-, m- or p-vinylbenzyl halide, will determine the position of the vinyl group in general formula VII. Hence when employing a mixture of e.g. m- and p-vinylbenzyl chloride, the ultimate product will be a mixture of compounds of general formula VII, having the vinyl group linked to the carbon atom in the 3- or 4-position of the aromatic ring.

During the metalation of compounds of general formulae IV, V and/or VI and the subsequent reaction with an electrophile, it is conceivable that more than one H on the α-carbon atom of R⁴ may be replaced with a metal atom. When said electrophile is a vinylbenzyl halide this may result in compounds of general formula 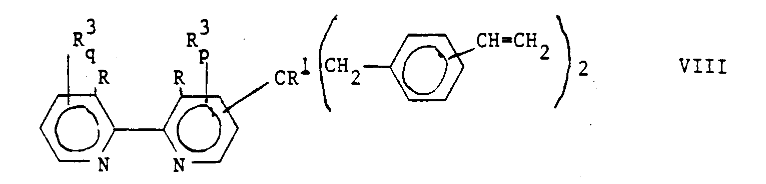
wherein R, R¹, R², R³, p and q have the same meanings as in general formula I. Hence in the preparation of compounds of general formula VII it is preferred, when R⁴ in general formula V is alkyl, that the α-carbon atom of R⁴ carries only one H and when R⁴ in general formula VI when R⁴ is alkyl, that the α-carbon atom carries only one H when R⁴ is linked to the carbon atom in the 2-position, and only two H's when R⁴ is linked to the carbon atom in the 4-position.

The compounds of general formulae VII and/or VIII may be polymerized by known techniques, as such or in the presence of one or more mono- or optionally polyethylenically unsaturated compounds, to provide polymers having at least one aryl group carrying a complex-forming moiety of general formula I. When the mixture of polymerizable compounds comprises only compounds of general formula VII and optionally one or more other monoethylenically unsaturated compounds, the resulting polymer will be a non-crosslinked homo- or copolymer. When however said mixture also includes one or more polyethylenically, preferably diethylenically unsaturated compounds, the resulting polymer will generally be a crosslinked polymer. Crosslinked complex-forming polymers may also be obtained when monoethylenically unsaturated compounds such as those of general formula VII, are polymerized in the presence of a compound of general formula VIII.

Suitable monoethylenically unsaturated compounds include monovinylaromatic compounds such as styrene and its analogs and homologs such as methylstyrenes, and ringsubstituted styrenes such as vinyltoluene; monoethylenically unsaturated carboxylic acids such as acrylic acid, methacrylic acid, itaconic acid, maleic acid, fumaric acid; esters of such monoethylenically unsaturated carboxylic acids such as: methyl acrylate, ethyl acrylate, butyl acrylate, 2-ethylhexyl acrylate, methyl methacrylate, 2-hydroxyethyl methacrylate and vinylpyridine.

Suitable diethylenically unsaturated compounds include compounds such as a divinylbenzene, a divinyltoluene, a divinylxylene, a divinylpyridine, divinyl ketone and divinylsulphone; divinyl ethers such as the divinyl ether of ethelene glycol, divinyl ethers of polyethylene glycols; diesters of two moles of a monoethylenically unsaturated carboxylic acid and a diol such as ethylene glycol diacrylate, ethylene glycol dimethacrylate and polyethylene glycol diacrylate. Divinylbenzene is a preferred diethylenically unsaturated compound.

The homo- and/or copolymerization of the compounds of general formula VII may be effected be contacting these compounds with e.g. a peroxy-type of radical initiator such as dibenzoylperoxide, cumenehydroperoxide, tert-butylperbenzoate, tert-butylhydroperoxide, peracetic acid and di-tert-butylperoxide, or with an azotype radical initiator such as α-α′-azobisisobutyronitrile (AIBN), 2,2′-azobis(2,4-methylvaleronitrile), 1,1′-azobis(cyclohexane-1-carbonitrile) and 2-α-naphthylazoisobutyronitrile. AIBN is a preferred azo-type radical initiator.

The polymerization of the hereinbefore mentioned compounds may also be effected in the presence of a redox type of initiator system. The polymerization will generally be conducted at a temperature in the range of from about 0 °C to about 150 °C. The amount of initiator or initiator system which may be employed in the present process is not critical and is generally in the range of from 0.01 to 10.0 %w on total weight of polymerizable compounds, preferably from 0.01 to 2.0 %w. The polymerization may be conducted e.g. in a homogeneous phase which includes both solution and bulk polymerization as well as in heterogeneous phase which includes emulsion and suspension polymerization. Suitable solvents which may be employed in the solution polymerization, which polymerization mode is a preferred mode, include aromatic hydrocarbons such as benzene, toluene, one or more xylenes; polar solvents such as acetone, methyl ethyl ketone, methyl isobutyl ketone and N,N-dimethylformamide.

An alternative and preferred process route for the preparation of polymers of the present invention is to react the metalated compounds of general formulae IV, V and/or VI with a halomethylated, preferably a chloromethylated polystyrene polymer. Suitable such polystyrenes include both polystyrene homo- and copolymers and also crosslinked polystyrene copolymers. The primary requirement for these polystyrene polymers is that they should be able to carry a halomethyl group, preferably a chloromethyl group. A preferred group of crosslinked polystyrene polymers are the polystyrene (PS)/- divinylbenzene (DVB) resins having a DVB content in the range of from about 0.5 to about 25 %w. PS/DVB resins are divided into two categories, i.e. the gel-type PS/DVB resins, having a DVB content which is generally not much higher than 2 to 3 %w, and the macroporous type of PS/DVB resins having a higher DVB content, generally ≧ 5 %w.

The halomethylation, more in particular the chloromethylation of the above described crosslinked resins is well known. Moreover, some types of chloromethylated PS/DVB resins are commercially available, e.g. gel-type chloromethylated PS/DVB resins which are also known as Merrifield resins.

The reaction between the hereinbefore mentioned metalated compounds of general formulae IV, V and/or VI and the halomethyl group carrying polymer may conveniently be conducted in the presence of an inert solvent such as diethyl ether or tetrahydrofuran, mixtures thereof, or mixtures thereof with a suitable hydrocarbon solvent and at a temperature in the range of from about -120 °C to about 100 °C.

The present invention also relates to the use of the hereinbefore described complex-forming polymers, which use is primarily related to their ability to form complexes with a wide range of metals or metal-containing compounds, i.e. to complexed polymers, their preparation, and use.

As the complex-forming moieties of these complex-forming polymers are either 2,2′-bipyridine or 1,10-phenanthroline type complex-forming moieties, it will be understood by those skilled in the art that these polymers may conveniently form complexes with the same metal or metal compounds which also form complexes with the corresponding monomeric bidentate 2,2′-bipyridine or 1,10-phenanthroline ligands; as well as with those metal or metal compounds which are able to form complexes with known complex-forming polymers, having similar types of complex-forming moieties as described in the hereinbefore cited documents. Suitable complex-forming metals and metal compounds have been outlined in US patent specification 3,810,888 and European patent application 0 045 277. Preferred metal compounds are those based on metals of group VIII and Ib of the periodic system.

The complexes may conveniently be prepared by contacting e.g. a solution of a complex-forming polymer with a solution of a suitable metal compound under complex-forming conditions. However when the complex-forming polymer is insoluble, as may be the case with a crosslinked complex-forming polymer, it may be advantageous to suspend said polymer in a solvent which may simultaneously act as a solvent for the metal compound or is at least (partially) miscible with the solution of the metal compound.

Although the obvious and preferred process route to arrive at complexed polymers of the present invention is to contact the complex-forming polymer with a suitable metal compound, as hereinbefore described, it is also possible to contact a compound of general formula VII with a suitable metal compound, and subsequently polymerize the resulting complex to arrive at a complexed polymer of the present invention. It is conceivable that this approach may be beneficial e.g. when aiming for crosslinked polymers wherein all possible complex-forming moieties have been complexed, which may not always be possible e.g. when contacting a suitable metal compound with a suspension of crosslinked complex-forming polymer. This method of complex-formation prior to polymerization may also be beneficial in the preparation of complexed polymers having more than one type of complex.

The complex-forming polymers of the present invention are versatile products which may be advantageously used in many applications, such as extraction and separation processes for metals and for metal compounds or in purification processes. Furthermore these polymers may be conveniently used in the form of their corresponding complexes with a metal or metal compound, as a catalyst or catalyst precursor for reactions such as hydrogenation, dehydrogenation, isomerization, hydroformylation and reductive carbonylation.

The invention will be further understood from the following examples.

### Preparation of 4-ethyl-1,10-phenanthroline

In a Slenck tube reactor equipped with a magnetic stirrer and in an inert atmosphere (argon) 2520µl 1.6 M (4.0 mmol) n-butyllithium solution in n-hexane was added dropwise with the aid of a syringe to a solution of 600 µl diisopropylamine (HDA) in 2800 µl anhydrous tetrahydrofuran (THF) at 0 °C. The resulting mixture was stirred for 15 minutes at 0 °C. To this mixture a solution of 749.0 mg (3.86 mmol) 4-methyl-1,10-phenanthroline, dissolved in 18 ml anhydrous THF, was added in 5 minutes. The reactor contents were stirred at 0 °C for 1 hour, whereupon 570 mg (4.0 mmol) methyl iodide was added with the aid of a syringe. The mixture was stirred at 0 °C for 45 minutes, after which the external cooling was stopped and reactor contents were allowed to warm to room temperature (18-20 °C) and stirred at that temperature for 18 hours. Subsequently 1 ml water was added to the reactor and the mixture was stirred for about 5 minutes and transferred into a separatory funnel with the aid of 15 ml water and 100 ml chloroform. After equilibration the organic phase was removed, and the aqueous phase was extracted with 50 ml chloroform. The combined chloroform phases were washed consecutively with 10 ml water and 15 ml brine, and dried over anhydrous magnesium sulphate. The chloroform was removed at 60 °C under reduced pressure (approx. 250 mbar) using a rotary evaporator. The crude product was purified by column chromatography using basic alumina; column diameter and height: 1.7 x 18 cm. After elution with 300 ml of diethyl ether the product was eluted with 200 ml chloroform. The appropriate fraction (yellow band) was collected and the solvent was removed at 60 °C under reduced pressure (approx. 250 mbar) using a rotary evaporator. The residue was sublimed at 120-180 °C under reduced pressure (0.5 mbar). The pale yellow solid was stored in a dessicator under reduced pressure (1 mbar) over phosphorus pentoxide.
Yield: 645.6 mg (3.1 mmol; 80.4%)

| Analysis for C₁₄H₁₂N₂: | | | |
|---|---|---|---|
| calculated | C 80.74% | H 5.80% | N 13.45% |
| found | C 80.54% | H 5.86% | N 13.42% |

### Example 1

### Preparation of a mixture of 4-[1-methyl-2-(3-vinylphenyl)]-ethyl-1,10-phenanthroline and 4-[1-methyl-2-(4-vinylphenyl)]-ethyl-1,10-phenanthroline

In a Slenck tube reactor equipped with a magnetic stirrer and in an inert atmosphere (argon) 2520µl 1.6 M (4.0 mmol) n-butyllithium solution in n-hexane was added dropwise with the aid of a syringe to a solution of 600 µl HDA in 2800 µl anhydrous THF at 0 °C. The resulting mixture was stirred for 15 minutes at 0 °C. To this mixture 799.0 mg (3.84 mmol) 4-ethyl-1,10-phenanthroline prepared following the hereinbefore described method and dissolved in 18 ml anhydrous THF was added in 5 minutes. The mixture was stirred at 0 °C for 2 hours, whereupon 604.8 mg (3.98 mmol) of an approx. 40:60 mixture of para- and meta-vinylbenzyl chloride was added with a syringe. Under continuous stirring the reactor contents were allowed to warm to room temperature (18-20 °C) for 30 minutes and, subsequently, heated for 2 hours at 65 °C, after which 1 ml of water was added to the reactor. After 5 minutes the reactor contents were cooled to room temperature and transferred into a separatory funnel with the aid of 30 ml water and 70 ml chloroform. After equilibration the organic phase was separated, and the aqueous phase was extracted with 70 ml chloroform. The combined organic phases were washed twice with 15 ml water, once with 20 ml brine, and dried over anhydrous magnesium sulphate. The solvent was removed at 60 °C under reduced pressure (approx. 250 mbar) using a rotary evaporator. The crude product was purified by column chromatography using basic alumina; column diameter and height: 1.7 x 20 cm. After elution with 300 ml of diethyl ether the product was eluted with 200 ml chloroform. The appropriate fraction (yellow band) was collected and the solvent was removed at 60 °C under reduced pressure (approx. 250 mbar) using a rotary evaporator leaving a brown oil. Yield: 1.07 g (3.3 mmol; 86%).

### Example 2

### Polymerization of products prepared in example 1.

A mixture of 400.8 mg (1.24 mmol) product as prepared in example 1, 7.7 mg azobisisobutyronitrile (AIBN) and 10 ml anhydrous benzene was introduced into a glass reactor equipped with a reflux condensor, gas inlet and a magnetic stirring device, and degassed three times. Subsequently the reactor contents heated at 75 °C for 24 hours in an argon atmosphere, after which period of time the reaction mixture was allowed to cool to room temperature. The mixture was diluted with 20 ml chloroform, filtered and concentrated to 4 ml at 70 °C under reduced pressure (approx. 250 mbar) using a rotary evaporator. Whilst stirring the residue was added dropwise to 40 ml diethyl ether. The resulting precipitate was redissolved in chloroform and purified by two further precipitations from methanol.
Yield: 95.3 mg (0.29 mmol; 24 %) pale brown solid.

| Analysis for (C₂₃H₂₀N₂)ₙ | | | |
|---|---|---|---|
| calculated | C 85.15% | H 6.21% | N 8.63% |
| found | C 84.43% | H 6.46% | N 8.54% |

### Example 3

### Preparation of copolymers based on products prepared in example 1 and styrene (method A)

A mixture of 463.8 mg (1.4 mmol) of the product as prepared in example 1, 245 mg (2.4 mmol) styrene, 6.5 mg AIBN, and 11.5 ml anhydrous benzene was introduced into similar reactor as in example 2 and degassed three times and, subsequently, heated at 75 °C for 24 hours in an argon atmosphere. Next the reaction mixture was allowed to cool to room temperature, and 10 ml chloroform was added. The mixture was concentrated to 2 ml at 80 °C under reduced pressure (approx. 250 mbar) using a rotary evaporator. Whilst stirring the residue was added dropwise to 40 ml diethyl ether/n-hexane (v/v = 1/1). The resulting precipitate was filtered off, redissolved in chloroform and purified by two further precipitations from diethyl ether/n-hexane (v/v = 1/1).
Yield: 175.5 mg pale brown solid.

| Analysis for (C₂₃H₂₀N₂)_{q}(C₈H₈)ᵣ with q = 0.29 and r = 0.71 | | | |
|---|---|---|---|
| calculated | C 88.28% | H 6.88% | N 4.84% |
| found | C 87.64% | H 7.06% | N 4.84% |

### Example 4

### Preparation of the reaction product of lithiated 4-ethyl-1,10-phenanthroline and chloromethylated polystyrene (Method B)

In a Slenck tube reactor equipped with a magnetic stirrer and in an inert atmosphere 315 µl 1.6 M (0.50 mmol) n-butyllithium solution in n-hexane was added dropwise with the aid of a syringe to a solution of 75 µl HDA in 300 µl anhydrous THF at 0 °C. The resulting mixture was stirred for 15 minutes at 0 °C, whereupon 100.6 mg (0.50 mmol) 4-ethyl-1,10-phenanthroline, prepared following the method as hereinbefore described and dissolved in 2.0 ml anhydrous THF, was added in 5 minutes. The mixture was stirred at 0 °C for 1 hour. After this period of time 200.7 mg chloromethylated polystyrene having a Cl content of approx. 5.1 %w - soluble chloromethylated polystyrene synthesized according to J. Gen. Chem. USSR, 38, 2546 (1968) and Helv. Chim. Acta, 56, 1214 (1973) - dissolved in 2.0 ml anhydrous THF, was added. The resulting mixture was allowed to warm to room temperature (18-20 °C) for 15 minutes and subsequently heated at 65 °C for 75 minutes. After the addition of 1 ml water and stirring for about 5 minutes the mixture was cooled to room temperature and transferred into a separatory funnel with the aid of 5 ml water and 50 ml chloroform.

The organic phase was separated and concentrated to 10 ml at 60 °C under reduced pressure (approx. 250 mbar) using a rotary evaporator. Whilst stirring the residue was added dropwise to a large excess of methanol. The resulting precipitate was filtered off, redissolved in 10 ml chloroform and purified by two further precipitations from methanol.
Yield: 121.1 mg pale brown solid.

| Analysis for (C₂₃H₂₀N₂)_{q}(C₈H₈)ᵣ(C₉H₉Cl)ₛ with q = 0.18, r = 0.82 and s = 0. | | | | |
|---|---|---|---|---|
| calculated: | C 89.39% | H 7.12% | N 3.51% | Cl 0.0% |
| found: | C 87.10% | H 6.76% | N 3.41% | Cl 0.28% |

In the examples hereinafter, which are related to the complexation of the polymers of the present invention with some metal compounds, the following abbreviations will be used:
- L :: polymer of the invention corresponding with one complex-forming moiety
- L¹:: L based on a polymer as prepared in example 2.
- L²:: L based on a polymer as prepared in example 3, but containing 62 %m styrene.
- L³:: L based on a polymer as prepared in example 3, but containing 91 %m styrene.
- Hd:: 1,5-hexadiene
- COT:: cyclooctene
- 2-Me-allyl:: 2-methylallyl
- COD:: 1,5-cyclooctadiene

### Example 5

### Preparation of Rh₂Cl₂(HCOO)₂(L¹)₂

A 100 ml autoclave (Hasteloy C) equipped with a magnetic stirring device was charged with 62 mg RhCl₂(2-Me-allyl), prepared according to H. Pasternak, E. Lancman, and F. Pruchnik. J. Mol. Catal., 29, 13 (1985); F. Pruchnik, Inorg. Nucl. Chem. Lett. 9, 1229 (1973), (0.27 mmol), 80 mg L¹ (0.25 mmol), 20 ml abs. ethanol and 4 ml 98% formic acid. The slurry in the autoclave was purged with argon. Subsequently, the autoclave was sealed and heated for 1 hour at 80 °C, followed by 5 hours at 110 °C with vigorous stirring. Next the autoclave was cooled to room temperature, opened, and a dark brown precipitate was filtered off, washed with ethanol and dried. 112 mg of product was isolated. Elemental analysis of said product revealed a complete complexation of the complex-forming moieties, indicating a yield of 89%.

### Examples 6 and 7

### Preparation of Rh₂Cl₂(HCOO)₂(L²)₂ and Rh₂Cl₂(HCOO)₂(L³)₂

Following the same procedure as in Example 5 the corresponding complexes with respectively L² and L³ were also prepared, employing the same molar ratio of RhCl₂(2-Me-allyl) to L. Elemental analysis revealed a complete complexation of the complex-forming moieties. Rh₂Cl₂(HCOO)₂(L²)₂ was isolated in a 64% yield and
Rh₂Cl₂(HCOO)₂(L³)₂ in a 83% yield

### Example 8

### Preparation of [Ir(Hd)(L³)]Cl

A solution of 40 mg (0.09 mmol) Ir(COT)₂Cl, prepared according to J.L. Herdé and C.V. Senoff, Inorg. Nucl. Chem. Lett., 7, 1029 (1971), in 3.5 ml degassed benzene was purged with argon in a 25 ml Slenck tube reactor equipped with a magnetic stirring device. Whilst stirring 0.30 ml 1,5-hexadiene was added to this solution and the resulting mixture was stirred at room temperature for 30 minutes, and filtered under argon. To the filtrate 143 mg L³ (corresponding with 0.10 mmol 1,10-phenanthroline moieties) dissolved in 2.0 ml degassed benzene was added under argon. The solution turned purple immediately, and was stirred for 2 hours under argon. The mixture was added to 10 ml diethyl ether and the resulting purple precipitate was filtered off and, after washing with diethyl ether and drying, 132 mg of product was isolated. Elemental analysis revealed a 86% complexation of the complex-forming moieties.

### Examples 9 and 10

### Preparation of [Ir(Hd)(L¹)]Cl and [Ir(Hd)(L²)]Cl

Following the same method as in Example 8, the corresponding complexes with respectively L¹ and L² were also prepared, employing the same molar ratio of Ir(COT)₂Cl to L. Elemental analysis of the resulting products revealed a complexation of 56 and 69% respectively, for [Ir(Hd)(L¹)]Cl and [Ir(Hd)(L²)]Cl.

### Example 11

### Preparation of [Rh(COD)(L²)]ClO₄

A solution of 83.6 mg L² (corresponding with 0.17 mmol 1,10-phenanthroline moieties) in 5.0 ml dichloromethane was added to a magnetically stirred solution of 40.0 mg [Rh(COD)ClO₄]₂, prepared according to R. Usón, L.A. Oro, D. Carmona, and M. Esteban, J. Organometal. Chem., 220, 103 (1981), (containing 0.17 mmol Rh) in 10 ml dichloromethane in an erlenmeyer flask at room temperature. The resulting mixture was stirred at room temperature for 30 minutes. Subsequently, 10 ml diethyl ether was added. The resulting orange-brown precipitate was filtered off, washed with diethyl ether and dried. 123.6 mg of product was collected. Elemental analysis showed complexation of 85%.

### Examples 12 and 13

### Preparation of [Rh(COD)(L¹)]ClO₄ and (Rh(COD)(L³))ClO₄

Following the same method as in Example 11 the corresponding complexes with respectively L¹ and L³ were also prepared, employing the same molar ratio of [Rh(COD)ClO₄]₂ to L. Elemental analysis of the resulting products showed a complexation of 83 and 82% respectively for [Rh(COD)(L¹)]ClO₄ and [Rh(COD)(L³)]ClO₄

### Example 14

### Preparation of 6-(1-methylbutyl)-2,2′-bipyridyl

In a Slenck tube reactor equipped with a magnetic stirrer and in an inert atmosphere (argon) 3.1 ml 1.6 N(4.7 mmol) n-butyllithium solution in n-hexane was added dropwise with the aid of a syringe to a solution of 700 µl HDA in 3500 µl anhydrous tetrahydrofuran (THF) at 0 °C. The resulting mixture was stirred for 20 minutes at 0 °C. To this mixture a solution of 931.9 mg (4.4 mmol) 6-(n-butyl)-2,2′-bipyridyl, prepared according to:
T. Kauffmann, J. König, and A. Woltermann, Chem. Ber., 109, 3864 (1976), dissolved in 7.0 ml anhydrous THF, was added in 5 minutes. The reactor contents were stirred at 0 °C for 1.5 hours, whereupon 661 mg (4.7 mmol) methyl iodide was added with the aid of a syringe. The mixture was stirred at 0 °C for 15 minutes, after which the external cooling was stopped and the reactor contents were allowed to warm to room temperature (18-20 °C) and stirred at that temperature for 1 hour. Subsequently, 1 ml water was added to the reactor and the mixture was stirred for about 5 minutes and transferred into a separatory funnel with the aid of 50 ml diethyl ether and 10 ml water. After equilibration the organic phase was removed, and the aqueous phase was extracted twice with 20 ml diethyl ether. The combined organic phases were washed with 10 ml brine, and dried over anhydrous magnesium sulphate. The organic solvents were removed at 70 °C at 1 bar and, subsequently, at reduced pressure (about 200 mbar) using a rotary evaporator. The crude product was purified by column chromatography using basic alumina (activity III); column diameter and height: 1.7 x 15 cm. The produkt was eluted with n-pentane/diethyl ether (3:2(v/v)). The appropriate fraction was collected and the solvents were removed at 70 °C under reduced pressure (about 200 mbar) using a rotary evaporator. The residue was distilled (short-path) at 100-130 °C under reduced pressure (1.5 mbar)
Yield: 880.0 mg (3.9 mmol 88.5%) colourless oil.

| Analysis for C₁₅H₁₈N₂: | | | |
|---|---|---|---|
| calculated | C 79.61% | H 8.02% | N 12.38% |
| found | C 79.73% | H 8.10% | N 12.12% |

### Preparation of a mixture of 6-[1-methyl-1-{(3-vinylphenyl)methyl}butyl]-2,2′-bipyridyl and 6-[1-methyl-1-{(4-vinylphenyl)methyl}butyl]-2,2′-bipyridyl.

In a Slenck tube reactor equipped with a magnetic stirrer and in an inert atmosphere (argon) 2.0 ml 1.5 N (3.0 mmol) n-butyllithium solution in n-hexane was added dropwise with the aid of a syringe to a solution of 440 µl HDA in 2100 µl anhydrous THF at 0 °C. The resulting mixture was stirred for 15 minutes at 0 °C. To this mixture 642.7 mg (2.84 mmol) 6-(1-methylbutyl)-2,2′-bipyridyl prepared following the hereinbefore described method and dissolved in 4.5 ml anhydrous THF was added in 5 minutes. The mixture was stirred at 0 °C for 1 hour, and subsequently, for 20 minutes at room temperature (18-20 °C), whereupon 464 mg (3.05 mmol) of an about 40:60 mixture of para- and metavinylbenzyl chloride was added with a syringe. The reactor contents were stirred at room temperature (18-20 °C) for 15 minutes and, subsequently, heated for 1.5 hours at 55 °C, after which 1 ml water was added to the reactor. After 5 minutes the reactor contents were cooled to room temperature and transferred into a separatory funnel with the aid of 20 ml water and 50 ml diethyl ether. After equilibration the organic phase was separated, and the aqueous phase was extracted with 25 ml diethyl ether. The combined organic phases were washed consecutively with 20 ml water and 20 ml brine, and dried over anhydrous magnesium sulphate. The solvents were removed at 70 °C at atmospheric pressure, and, subsequently, at reduced pressure (about 250 mbar) using a rotary evaporator. The crude product was purified by column chromatography using basic alumina (activity III); column diameter and height: 1.7 x 23 cm. After elution with 550 ml n-pentane the product was eluted with n-pentane/diethyl/ether [9:1 (v/v)]. The appropriate fraction was collected and the solvent was removed at 70 °C at reduced pressure (about 250 mbar), using a rotary evaporator.
Yield: 432.6 mg (1.3 mmol; 45%) colourless oil.

### Example 15

### Preparation of the reaction product of lithiated 6-(1-methylbutyl)-2,2′-bipyridyl and chloromethylated polystyrene (Method B)

In a Slenck tube reactor equipped with a magnetic stirrer and in an inert atmosphere (argon) 420 µl 1.5 N (0.63 mmol) n-butyllithium solution in n-hexane was added dropwise with the aid of a syringe to a solution of 90 µl HDA in 420 µl anhydrous THF at 0 °C. The resulting mixture was stirred for 25 minutes at 0 °C, whereupon 125.5 mg (0.56 mmol) 6-(1-methylbutyl)-2,2′-bipyridyl prepared following the hereinbefore described method and dissolved in 1.3 ml anhydrous THF was added in 5 minutes. The mixture was stirred at 0 °C for 45 minutes and, subsequently, for 25 minutes at room temperature (18-20 °C). After this period of time 250.3 mg chloromethylated polystyrene, having a Cl content of about 5.1%, dissolved in 2.5 mol anhydrous THF was added. The soluble chloromethylated polystyrene was synthesized according to J. Gen. Chem. USSR, 38, 2546 (1968) and Helv. Chim. Acta, 56, 1214 (1973). The resulting mixture was stirred at room temperature for 45 minutes and, subsequently, heated at 50 °C for 3 hours. After the addition of 200 µl water and stirring for about 5 minutes the mixture was cooled to room temperature and transferred into a separatory funnel with the aid of 20 ml water and 50 ml diethyl ether. After equilibration the organic phase was separated, washed once with 20 ml water, dried over anhydrous magnesium sulphate, and concentrated to about 2 ml at 70 °C at atmospheric pressure using a rotary evaporator. Whilst stirring the residue was added dropwise to a large excess of methanol. The resulting precipitate was filtered off, redissolved in 2 ml diethyl ether and purified by two further precipitations from methanol. The precipitate was dried over phosphorus pentoxide using a dynamic vacuum (1.3 mbar) for 6 hours.
Yield: 301.5 mg pale yellow solid.

| Analysis for (C₂₄H₂₆N₂)_{q}(C₈H₈)ᵣ(C₉H₉Cl)ₛ with q = 0.21, r = 0.78, s = 0.01. | | | | |
|---|---|---|---|---|
| calculated | C 88.28% | H 7.68% | N 3.80% | Cl 0.23% |
| found | C 88.04% | H 7.85% | N 3.82% | Cl 0.29% |

### Example 16

### Preparation of 2-(1-methylbutyl)-1,10-phenanthroline

In a Slenck tube reactor equipped with a magnetic stirrer and in an inert atmosphere (argon) 3.1 ml 1.5 N (4.65 mmol) n-butyllithium solution in n-hexane was added dropwise with the aid of syringe to a solution of 700 µl HDA in 3500 µl anhydrous THF at 0 °C. The resulting mixture was stirred for 1 hour at 0 °C. To this mixture 1034.4 mg (4.4 mmol) 2-(n-butyl)-1,10-phenanthroline, prepared according to: P.J. Pijper, H. van der Goot, H. Timmerman, and W.Th. Nauta, Eur. J. Med. Chem. Chim. Ther., 19, 399 (1984), dissolved in 9.0 ml anhydrous THF was added in 5 minutes. The reactor contents were stirred at 0 °C for 2 hours, whereupon 661 mg (4.7 mmol) methyl iodide was added with the aid of a syringe.

The resulting mixture was stirred for 15 minutes at 0 °C and, subsequently, for 23 hours at room temperature (18-20 °C). Subsequently, 1 ml water was added to the reactor and the mixture was stirred for about 5 minutes and transferred into a separatory funnel with the aid of 50 ml water and 100 ml diethyl ether. After equilibration the organic phase was removed, and the aqueous phase was extracted with 100 ml diethyl ether. The combined organic phases were washed consecutively with 20 ml water and 20 ml brine, and dried over anhydrous magnesium sulphate. The solvents were removed at 70 °C at atmospheric pressure, and, subsequently at reduced pressure (about 250 mbar) using a rotary evaporator. The residue was (short path) distilled at 85-160 °C under reduced pressure (0.04 mbar).
Yield: 1001.5 mg (4.0 mmol; 91%) bright yellow viscous liquid.

| Analysis for C₁₇H₁₈N₂: | | | |
|---|---|---|---|
| calculated | C 81.56% | H 7.25% | N 11.19% |
| found | C 81.55% | H 7.28% | N 11.09% |

### Preparation of a mixture of 2-[1-methyl-1-{(3-vinylphenyl)methyl}butyl]-1,10-phenanthroline and 2-[1-methyl-1-{(4-vinylphenyl)methyl}butyl]-1,10-phenanthroline.

In a Slenck tube reactor equipped with a magnetic stirrer and in an inert atmosphere (argon) 670 µl 1.5 N (1.0 mmol) n-butyllithium solution in n-hexane was added dropwise with the aid of a syringe to a solution of 150 µl HDA in 700 µl anhydrous THF at 0 °C. The resulting mixture was stirred for 20 minutes at 0 °C. To this mixture 230.4 mg (0.92 mmol) 2-(2-pentyl)-1,10-phenanthroline prepared following the hereinbefore described method and dissolved in 8.0 ml anhydrous THF was added in 5 minutes. The mixture was stirred for 15 minutes at 0 °C and, subsequently, for 4 hours at room temperature (18-20 °C). After this period of time 162 mg (1.06 mmol) of an about 40:60 mixture of para- and meta-vinylbenzyl chloride was added with a syringe. The contents of the reactor were stirred at room temperature for 19 hours and, subsequently, for 5 hours at 45 °C, after which 1 ml water was added to the reactor. After 5 minutes the reactor contents were cooled to room temperature and transferred into a separatory funnel with the aid of 25 ml water and 50 ml dichloromethane. After equilibration the organic phase was separated, and the aqueous phase was extracted with 50 ml of dichloromethane. The combined organic phases were washed consecutively with 15 ml water and 15 ml brine, and dried over anhydrous magnesium sulphate. The solvents were removed at 70 °C at atmospheric pressure and, subsequently, at reduced pressure (about 250 mbar) using a rotary evaporator. The residue was separated by column chromatography using kieselgel 60 (80 g); column diameter and height: 1.7 x 20 cm. Elution was performed using diethyl ether/n-pentane [1/1 (v/v)] as eluens. The appropriate fraction was collected and the solvent was removed at 70 °C at atmospheric pressure using a rotary evaporator.
- Yield:: 143.3 mg (0.4 mmol; 59% on the basis of converted starting material) pale yellow oil.
Using the same eluens 63.4 mg (28% recovery) of starting material was isolated in a fraction subsequent to the fraction containing the desired product mixture.

### Example 17

### Preparation of the reaction product of lithiated 2-(n-butyl)-1,10-phenanthroline and (crosslinked) chloromethylated polystyrene (Method B)

In a Slenck tube reactor equipped with a magnetic stirrer and in an inert atmosphere (argon) 670 µl 1.5 N (1.0 mmol) n-butyllithium solution in n-hexane was added dropwise with the aid of a syringe to a solution of 150 µl HDA in 700 µl anhydrous THF at 0 °C. The resulting mixture was stirred for 20 minutes at 0 °C, whereupon 222.3 mg (0.094 mmol) 2-(n-butyl)-1,10-phenanthroline - prepared according to: P.J. Pijper, H. van der Goot, H. Timmerman, and W.Th. Nauta, Eur. J. Med. Chem. - Chim. Ther., 19, 399 (1984), dissolved in 7.0 ml anhydrous THF was added in 5 minutes. The mixture was stirred for 1.75 hours at 0 °C. After this period of time 679.2 mg chloromethylated polystyrene (crosslinked by 2% divinylbenzene) 200-400 mesh purified by consecutive washings with water/dioxane [1/1 (v/v)], water/dioxane/conc. hydrochloric acid [4/5/1 (v/v/v)], dioxane, methanol, and soxhlet extraction with anhydrous THF for 18 hours; dried by evacuating over phosphorus pentoxide for 24 hours at 90 °C using a dynamic vacuum (about 0.1 mbar); elemental analysis: C: 90.43%; H: 7.68%; Cl: 1.72%) was added. The resulting mixture was heated at 70 °C for 18 hours. After the addition of 0.5 ml water and stirring for 1.5 hours the mixture was cooled to room temperature and the solids were filtered off. The solids were purified by consecutive washings with dioxane, dioxane/water [1/1 (v/v)], diethyl ether, THF, and methanol, and subsequent soxhlet extraction with anhydrous THF for 18 hours, followed by consecutive washings with diethyl ether, chloroform, methanol, n-hexane, and diethyl ether. The solids were dried over phosphorus pentoxide for 21 hours at 80 °C under reduced pressure (about 0.1 mbar) using a dynamic vacuum.
Yield: 615.3 mg green solid

| | | | | |
|---|---|---|---|---|
| Elemental analysis: | C: 90.41% | H 7.65% | Cl: <0.10% | N: 1.42% |

## Claims

1. A polymer having at least one pendant aryl group carrying a complex-forming moiety of general formula 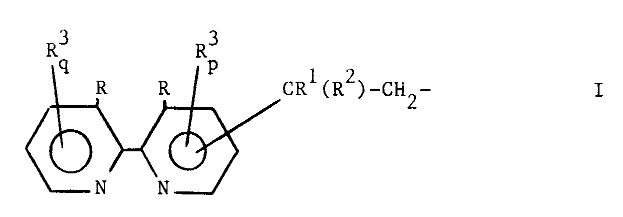 wherein each R individually is H, or both R's together form a group -CH=CH-; R¹ is H or an alkyl group; R² is H, an alkyl or a vinylbenzyl group or a group 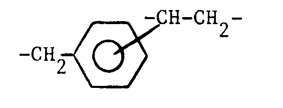 wherein the -CH-CH₂- moiety forms part of macromolecular polymer chain; or both R¹ and R² together form a group -(CH₂)ₙ- wherein n is an integer in the range of from 2 up to and including 6; each R³ individually is an alkyl, phenyl, alkoxy, phenoxy, alkylthio or phenylthio group; p and q are integers from 0 up to and including 2, and from 0 up to and including 3, respectively; the -CR¹(R²)-CH₂- group is linked to a carbon atom of the heterocyclic aromatic ring which occupies the ortho- or para-position with respect to the nitrogen atom in said heterocyclic ring, with the proviso that at least one of R¹ or R² represents H if both R's together form a group -CH=CH- and if at the same time the group -CR¹(R²)-CH₂- occupies the para-position with respect to the nitrogen atom in said heterocyclic aromatic ring.

2. A polymer according to claim 1, wherein in general formula I each R individually is H.

3. A polymer according to claim 1, wherein in general formula I both R's together form a group -CH=CH-.

4. A polymer according to any of the preceding claims, wherein in general formula I R¹ is an alkyl group having from 1 to 6 carbon atoms.

5. A polymer according to any of the preceding claims, wherein in general formula I R² is an alkyl group having from 1 to 6 carbon atoms.

6. A polymer according to any of the preceding claims, wherein in general formula I the integers p and q are 0.

7. A polymer according to any of the preceding claims, wherein said polymer is a homopolymer.

8. A polymer according to any of claims 1 to 6, wherein said polymer is a copolymer.

9. A copolymer according to claim 8, which is a copolymer of a monovinylaromatic compound and a divinyl unsaturated compound.

10. A copolymer according to claim 9, which is a polystyrene/divinylbenzene copolymer.

11. A polystyrene/divinylbenzene copolymer according to claim 10, wherein divinylbenzene is present in an amount which corresponds with 0.5 to 25 %w of said copolymer.

12. A process for the preparation of a polymer according to any of the preceding claims, wherein a compound of general formula 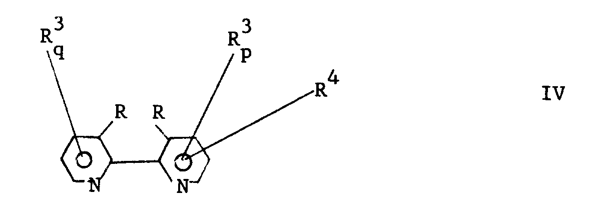 in which formula R, R³, p and q have the same meaning as in general formula I provided that R³ is essentially inert vis-à-vis the metalating agent defined hereinafter and R⁴ is an alkyl group, or a cycloaliphatic group having from 3 to 7 carbon atoms in the ring, and is linked to a carbon atom of the heterocyclic aromatic ring, which occupies the ortho- or para-position with respect to the nitrogen atom in said heterocyclic aromatic ring and which alkyl group or cycloaliphatic group has at least one hydrogen on the alpha-carbon atom with the proviso that R⁴ is an alkyl group having at least two hydrogens on the alpha-carbon atom, when both R's together form a group -CH=CH- and R⁴ is linked to the carbon atom which occupies the para-position to the nitrogen atom in said heterocyclic aromatic ring, is contacted with a metalating agent followed by reaction of the metalated compound with either:
A.
1. at least one vinylbenzyl halide and subsequently
2. polymerizing the reaction product of A.1. or
B. a halomethylated polystyrene.

13. A process according to claim 12, wherein R⁴ is an alkyl group having from 1 to 6 carbon atoms.

14. A process according to claim 12 or 13, wherein the metalating agent is an alkali metal amide.

15. A process according to claim 14, wherein the alkali metal amide is lithiumdiisopropylamide.

16. A process according to any of claims 12 to 15, wherein the vinylbenzyl halide is a vinylbenzyl chloride.

17. A process according to any of claims 12 to 16, wherein the reaction product of A.1 is copolymerized in the presence of at least one monoethylenically unsaturated compound and/or at least one polyethylenically unsaturated compound.

18. A process according to claim 17, wherein the polyethylenically unsaturated compound is divinylbenzene.

19. A process according to claim 18, wherein divinylbenzene is present in an amount which corresponds with about 0.5 to about 25 %w of total weight of polymerizable compounds.

20. A process according to any of claims 12 to 15, wherein the halomethylated polystyrene is a chloromethylated polystyrene.

21. A process according to claim 20, wherein the chloromethylated polystyrene is a crosslinked chloromethylated polystyrene.

22. A process according to claim 21, wherein the crosslinked chloromethylated polystyrene is a chloromethylated polystyrene/divinylbenzene copolymer, wherein divinylbenzene is present in an amount which corresponds with about 0.5 to about 25 %w of the polystyrene/divinylbenzene copolymer.

23. Complexed polymers prepared by contacting a solution of a polymer according to any of claims 1 to 11 with a metal compound under complex-forming conditions.

24. Complexed polymers according to claim 23, wherein the metal compounds are based on metals of group VIII and 1b of the Periodic Table of the Elements.

## Patentansprüche

1. Ein Polymer mit mindestens einer daran hängenden Arylgruppe mit einer komplexbildenden Gruppe der allgemeinen Formel 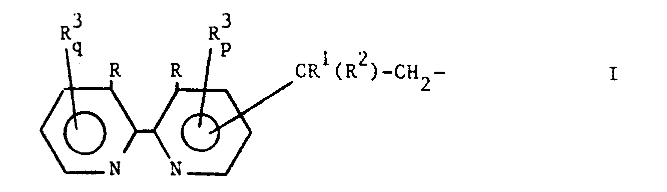 in welcher jedes R jeweils H ist oder beide Gruppen R zusammen eine Gruppe -CH=CH- bilden; R¹ H oder eine Alkylgruppe ist; R² H, eine Alkylgruppe oder eine Vinylbenzylgruppe oder eine Gruppe 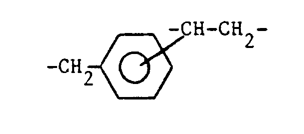 ist,
in welcher die 〉CH-CH₂-Gruppe einen Teil der makromolekularen Polymerkette bildet: oder beide Gruppen R¹ und R² zusammen eine Gruppe -(CH₂)n- bilden, in welcher n eine ganze Zahl im Bereich von 2 bis einschließlich 6 ist; jede Gruppe R³ unabhängig eine Alkyl-, Phenyl-, Alkoxy-, Phenoxy-, Alkylthio- oder Phenylthiogruppe ist; p und q ganze Zahlen von 0 bis einschließlich 2 bzw. von 0 bis einschließlich 3 sind; die Gruppe -CR¹(R²)-CH₂- an ein Kohlenstoffatom des heterocyclischen aromatischen Rings gebunden ist, das in der ortho- oder para-Stellung in bezug auf das Stickstoffatom in dem genannten heterocyclischen Ring vorliegt, mit der Maßgabe, daß mindestens eine der Gruppen R¹ und R² H darstellt, wenn beide Gruppen R zusammen eine Gruppe -CH=CH- bilden und wenn gleichzeitig die Gruppe -CR¹(R²)-CH₂- in der para-Stellung in bezug auf das Stickstoffatom in dem genannten heterocyclischen aromatischen Ring vorliegt.

2. Ein Polymer nach Anspruch 1, wobei in der allgemeinen Formel I die Gruppe R jeweils H darstellt.

3. Ein Polder nach Anspruch 1, wobei in der allgemeinen Formel I beide Gruppen R zusammen eine Gruppe -CH=CH- bilden.

4. Ein Polymer nach einem der vorstehenden Ansprüche, wobei in der allgemeinen Formel I R¹ eine Alkylgruppe mit 1 bis 6 Kohlenstoffatomen ist.

5. Ein Polymer nach einem der vorstehenden Ansprüche, wobei in der allgemeinen Formel I R² eine Alkylgruppe mit 1 bis 6 Kohlenstoffatomen ist.

6. Ein Polymer nach einem der vorstehenden Ansprüche, wobei in der allgemeinen Formel I die ganzen Zahlen p und q den Wert 0 haben.

7. Ein Polymer nach einem der vorstehenden Ansprüche, wobei das genannte Polymer ein Homopolymer ist.

8. Ein Polymer nach einem der Ansprüche 1 bis 6, wobei das genannte Polymer ein Copolymer ist.

9. Ein Copolymer nach Anspruch 8, das ein Copolymer einer monovinylaromatischen Verbindung und einer divinylungesättigten Verbindung ist.

10. Ein Copolymer nach Anspruch 9, welches ein Polystyrol/Divinylbenzolcopolymer ist.

11. Ein Polystyrol/Divinylbenzolcopolymer nach Anspruch 10, wobei das Divinylbenzol in einer Menge vorliegt, die 0,5 bis 25 Gewichtsprozent des genannten Copolymers entspricht.

12. Ein Verfahren zur Herstellung eines Polymers nach einem der vorstehenden Ansprüche, in welchem eine Verbindung der allgemeinen Formel 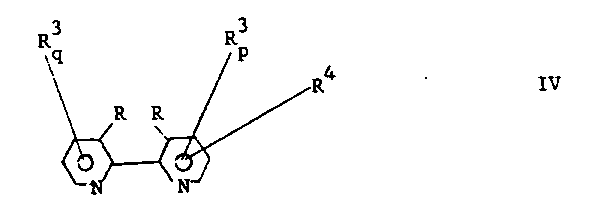 in welcher R, R³, p und q wie in der allgemeinen Formel I definiert sind, vorausgesetzt, daß R³ im wesentlichen inert gegenüber dem nachstehend definierten metallierenden Mittel ist, und R⁴ eine Alkylgruppe oder eine cycloaliphatische Gruppe mit 3 bis 7 Kohlenstoffatomen im Ring ist und an ein Kohlenstoffatom des heterocyclischen aromatischen Rings gebunden ist, das in der ortho- oder para-Stellung in bezug auf das Stickstoffatom in dem genannten heterocyclischen aromatischen Ring vorliegt, und welche Alkylgruppe oder cycloaliphatische Gruppe mindestens ein Wasserstoffatom am alpha-Kohlenstoffatom aufweist, mit der Maßgabe, daß R⁴ eine Alkylgruppe mit mindestens 2 Wasserstoffatomen am alpha-Kohlenstoffatom ist, wenn beide Gruppen R zusammen eine Gruppe -CH=CH- bilden, und R⁴ an das Kohlenstoffatom gebunden ist, das in der para-Stellung zum Stickstoffatom in dem genannten heterocyclischen aromatischen Ring vorliegt, mit einem metallierenden Mittel kontaktiert wird, worauf die Umsetzung der metallmodifizierten Verbindung mit entweder
A.
1. mindestens einem Vinylbenzylhalogenid und anschließendem
2. Polymerisieren des Reaktionsprodukts von A.1 oder
B. einem halogenmethylierten Polystyrol
folgt.

13. Ein Verfahren nach Anspruch 12, in welchem R⁴ eine Alkylgruppe mit 1 bis 6 Kohlenstoffatomen ist.

14. Ein Verfahren nach Anspruch 12 oder 13, in welchem das metallierende Mittel ein Alkalimetallamid ist.

15. Ein Verfahren nach Anspruch 14, in welchem das Alkalimetallamid Lithiumdiisopropylamid ist.

16. Ein Verfahren nach einem der Ansprüche 12 bis 15, in welchem das Vinylbenzylhalogenid Vinylbenzylchlorid ist.

17. Ein Verfahren nach einem der Ansprüche 12 bis 16, in welchem das Reaktionsprodukt von A.1 in Gegenwart mindestens einer monoethylenisch ungesättigten Verbindung und/oder mindestens einer polyethylenisch ungesättigten Verbindung copolymerisiert wird.

18. Ein Verfahren nach Anspruch 17, in welchem die polyethylenisch ungesättigte Verbindung Divinylbenzol ist.

19. Ein Verfahren nach Anspruch 18, in welchem Divinylbenzol in einer Menge vorliegt, die ca. 0,5 bis ca. 25 Gewichtsprozent des Gesamtgewichts der polymerisierbaren Verbindungen entspricht.

20. Ein Verfahren nach einem der Ansprüche 12 bis 15, in welchem das halogenmethylierte Polystyrol ein chlormethyliertes Polystyrol ist.

21. Ein Verfahren nach Anspruch 20, in welchem das chlormethylierte Polystyrol ein vernetztes chlormethyliertes Polystyrol ist.

22. Ein Verfahren nach Anspruch 21, in wlchem das vernetzte chlormethylierte Polystyrol ein chlormethyliertes Polystyrol/Divinylbenzolcopolymer ist, wobei das Divinylbenzol in einer Menge vorliegt, die ca. 0,5 bis 25 Gewichtsprozent des Polystyrol/Divinylbenzolcopolymers entspricht.

23. Komplexierte Polymere, hergestellt durch Kontaktieren einer Lösung eines Polymers nach einem der Ansprüche 1 bis 11 mit einer Metallverbindung unter Komplex-Bildungs-Bedingungen.

24. Zu Komplexen gebildete Polymere nach Anspruch 23, wobei die Metallverbindungen auf Metallen der Gruppe VIII und 1b des Periodischen Systems der Elemente basieren.

## Revendications

1. Polymère ayant au moins un groupe aryle pendant portant une partie complexante de formule générale 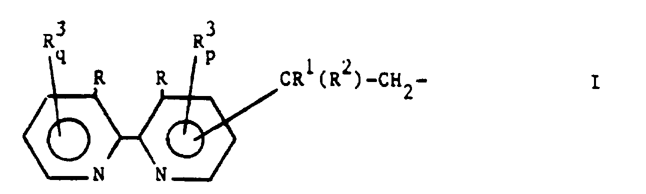 dans laquelle chaque R représente individuellement un atome H, ou bien les deux R forment ensemble un groupe -CH=CH-; R¹ représente un atome H ou un groupe alkyle; R² représente un atome H, un groupe alkyle ou un groupe vinylbenzyle ou un groupe 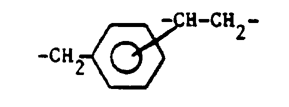 dans lequel la fraction -CH-CH₂ forme une partie d'une chaîne polymère macromoléculaire; ou bien les deux R¹ et R² forment ensemble un groupe -(CH₂)ₙ- dans lequel n est un nombre entier dans la gamme de 2 jusqu'à 6 inclus; chaque R³ représente individuellement un groupe alkyle, phényle, alkoxy, phénoxy, alkylthio ou phénylthio; p et q sont des nombres entiers de 0 jusqu'à 2 inclus, et de 0 jusqu'à 3 inclus, respectivement; le groupe -CR¹(R²)-CH₂- est lié à un atome de carbone du noyau aromatique hétérocyclique qui occupe la position ortho ou para par rapport à l'atome d'azote dans ledit noyau hétérocyclique, à condition qu'au moins un groupe R¹ ou R² représente un atome H si les deux R forment ensemble un groupe -CH=CH- et si au même moment, le groupe -CR¹(R²)-CH₂- occupe la position para par rapport à l'atome d'azote dans ledit noyau hétérocyclique.

2. Polymère selon la revendication 1, dans lequel dans la formule générale I, chaque R représente individuellement un atome H.

3. Polymère selon la revendication 1, dans lequel dans la formule générale I, les deux R forment ensemble un groupe -CH=CH-.

4. Polymère selon l'une quelconque des revendications précédentes, dans lequel dans la formule générale I, R¹ représente un groupe alkyle ayant de 1 à 6 atomes de carbone.

5. Polymère selon l'une quelconque des revendications précédentes, dans lequel dans la formule générale I, R² représente un groupe alkyle ayant de 1 à 6 atomes de carbone.

6. Polymère selon l'une quelconque des revendications précédentes, dans lequel dans la formule générale I, les nombres entiers p et q valent 0.

7. Polymère selon l'une quelconque des revendications précédentes, dans lequel ledit polymère est un homopolymère.

8. Polymère selon l'une quelconque des revendications 1 à 6, dans lequel ledit polymère est un copolymère.

9. Copolymère selon la revendication 8, qui est un copolymère d'un composé monovinylaromatique et d'un composé insaturé divinyle.

10. Copolymère selon la revendication 9, qui est un copolymère de polystyrène/divinylbenzène.

11. Copolymère de polystyrène/divinylbenzène selon la revendication 10, dans lequel le divinylbenzène est présent en une quantité qui correspond de 0,5 à 25% en poids dudit copolymère.

12. Procédé pour la préparation d'un polymère selon l'une quelconque des revendications précédentes, dans lequel un composé de formule générale 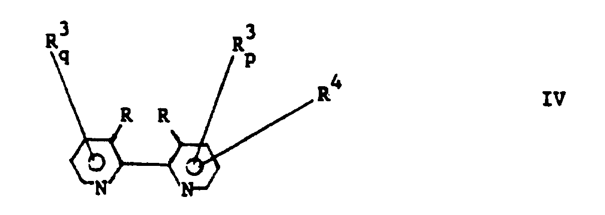 dans laquelle R, R³, p et q ont la même signification que dans la formule générale I, à condition que R³ soit essentiellement inerte vis-à-vis de l'agent de métalation défini ci-après et R⁴ est un groupe alkyle ou un groupe cycloaliphatique ayant de 3 à 7 atomes de carbone dans le noyau, et est lié à un atome de carbone du noyau aromatique hétérocyclique, qui occupe la position ortho ou para par rapport à l'atome d'azote dans ledit noyau hétérocyclique et groupe alkyle ou groupe cycloaliphatique qui possède au moins un atome d'hydrogène sur l'atome de carbone en alpha à condition que R⁴ soit un groupe alkyle ayant au moins deux atomes d'hydrogène sur l'atome de carbone en alpha, quand les deux R forment ensemble un groupe -CH=CH- et R⁴ est lié à l'atome de carbone qui occupe la position para par rapport à l'atome d'azote dans ledit noyau hétérocyclique, est mis en contact avec un agent de métalation, suivi de la réaction du composé métalé avec soit:
A.
1. au moins un halogénure de vinylbenzyle et ultérieurement
2. la polymérisation du produit réactionnel de A.1. ou
B. un polystyrène halogénométhylé.

13. Procédé selon la revendication 12, dans lequel R⁴ est un groupe alkyle ayant de 1 à 6 atomes de carbone.

14. Procédé selon la revendication 12 ou 13, dans lequel l'agent de métalation est un amide de métal alcalin.

15. Procédé selon la revendication 14, dans lequel l'amide de métal alcalin est un diisopropylamide-lithium.

16. Procédé selon l'une quelconque des revendications 12 à 15, dans lequel l'halogénure de vinylbenzyle est un chlorure de vinylbenzyle.

17. Procédé selon l'une quelconque des revendications 12 à 16, dans lequel le produit réactionnel de A.1 est copolymérisé en présence d'au moins un composé insaturé monoéthyléniquement et/ou au moins un composé insaturé polyéthyléniquement.

18. Procédé selon la revendication 17, dans lequel le composé insaturé polyéthyléniquement est un divinylbenzène.

19. Procédé selon la revendication 18, dans lequel le divinylbenzène est présent en une quantité qui correspond d'environ 0,5 à environ 25% en poids du poids total des composés polymérisables.

20. Procédé selon l'une quelconque des revendications 12 à 15, dans lequel le polystyrène halogénométhylé est un polystyrène chlorométhylé.

21. Procédé selon la revendication 20, dans lequel le polystyrène chlorométhylé est un polystyrène chlorométhylé réticulé.

22. Procédé selon la revendication 21, dans lequel le polystyrène chlorométhylé réticulé est un copolymère de polystyrène/divinylbenzène chlorométhylé, dans lequel le divinylbenzène est présent en une quantité qui correspond d'environ 0,5 à environ 25% en poids du copolymère de polystyrène/divinylbenzène.

23. Polymères complexés préparés par la mise en contact d'une solution d'un polymère selon l'une quelconque des revendications 1 à 11 avec un composé métallique dans des conditions complexantes.

24. Polymères complexés selon la revendication 23, dans lesquels les composés métalliques sont basés sur des métaux des groupes VIII et Ib du tableau périodique des éléments.
